# EUROPÄISCHE PATENTSCHRIFT

(11) **EP 0 964 847 B1**
(45) Veröffentlichungstag und Bekanntmachung des Hinweises auf die Patenterteilung: **02.01.2002**
(21) Anmeldenummer: 98905374.9
(22) Anmeldetag: 30.01.1998
(51) Int. Cl.: C07C 209/82, C07C 211/09

(54) **VERFAHREN ZUR ABTRENNUNG EINES IMINS AUS EINER MISCHUNG ENTHALTEND EIN AMIN UND EIN IMIN**
METHOD FOR SEPARATING AN IMINE FROM A MIXTURE CONTAINING AN AMINE AND AN IMINE
PROCEDE POUR SEPARER UNE IMINE COMPRISE DANS UN MELANGE CONTENANT UNE AMINE ET UNE IMINE

(30) Priorität: 07.02.1997 DE 19704614
(43) Veröffentlichungstag der Anmeldung: 22.12.1999
(73) Patentinhaber: BASF AKTIENGESELLSCHAFT, 67056 Ludwigshafen (DE)
(72) Erfinder: LUYKEN, Hermann, D-67069 Ludwigshafen (DE); BASSLER, Peter, D-68519 Viernheim (DE); REHFINGER, Alwin, D-67112 Mutterstadt (DE)
(86) Internationale Anmeldenummer: EP9800504
(87) Internationale Veröffentlichungsnummer: WO9834900

(56) Entgegenhaltungen:
- WO-A-96/20931

## Beschreibung

Die vorliegende Erfindung betrifft ein Verfahren zur destillativen Abtrennung eines Teiles oder der Gesamtheit eines Imins (III), ausgewählt aus der Gruppe bestehend aus Aminohexylidenimin, Tetrahydroazepin oder deren Addukte, aus einer Mischung (II) enthaltend ein Amin (I), ausgewählt aus der Gruppe bestehend aus Hexamethylendiamin und 6-Aminocapronitril und Imin (III), dadurch gekennzeichnet, daß man dem Destillationsgemisch eine gegenüber dem Amin (I) unter den Destillationsbedingungen inerte Verbindung (IV), deren Siedepunkt unter den Destillationsbedingungen über dem Siedepunkt des Amins (I) liegt, zugibt und nach der Destillation eine Mischung (VI) als Sumpfprodukt enthaltend im wesentlichen eine Verbindung (IV) erhält.

Mischungen enthaltend ein Amin und ein Imin fallen üblicherweise bei der Hydrierung von Nitrilen zu Aminen an.

Die vollständige Hydrierung von Adipodinitril zu Hexamethylendiamin, sowie die partielle Hydrierung unter gleichzeitiger Herstellung von Hexamethylendiamin und 6-Amincapronitril, in Gegenwart eines Katalysators auf der Basis eines Metalls wie Nickel, Cobalt, Eisen, Rhodium oder Ruthenium ist allgemein bekannt, beispielsweise aus: K.Weissermel, H.-J.Arpe, Industrielle Organische Chemie, 3.Auflage, VCH Verlagsgesellschaft mbH, Weinheim, 1988, Seite 266, US-A 4 601 859, US-A 2 762 835, US-A 2 208 598, DE-A 848 654, DE-A 954 416, DE-A 42 35 466, US-A 3 696 153, DE-A 19500222, WO 92/21650 und der Deutschen Anmeldung 19548289.1.

Als Nebenprodukte entstehen unter anderem Imine wie Aminohexylidenimin und Tetrahydroazepin.

Diese Imine, die wegen ihrer Farbgebung und Verschlechterung der Produkteigenschaften unerwünschte Verunreinigungen der üblicherweise für die Herstellung von Kunstfasern verwendeten Amine darstellen, lassen sich nur mit erheblichem Aufwand von den Aminen abtrennen.

So ist aus GB-A-893 709 bekannt, einen Verweilzeitbehälter in die Rückflußleitung einer für die Reinigung von Hexamethylendiamin verwendeten Destillationskolonne einzubauen.

GB-A-1 238 351 beschreibt die Abtrennung von Hexamethylendiamin aus Mischungen, die Hexamethylendiamin und Imine enthalten, durch Zugabe von Akalihydroxid-Gemischen.

Aus GB-A-1 041 442 ist bekannt, in eine für die Abtrennung von Hexamethylendiamin aus Mischungen, die Hexamethylendiamin und Imine enthalten, verwendete Destillationskolonne während der Destillation Kohlendioxid einzuleiten.

Nachteilig bei den genannten Verfahren sind die Anwendung großer Behälter mit einer verschlechterten Regelbarkeit der Destillationskolonnen und die Bildung von Feststoffen, die zu Verstopfungen führen können.

Aufgabe der vorliegenden Erfindung war es daher, ein Verfahren bereitzustellen, das die Abtrennung eines Imins aus Mischungen enthaltend ein Amin und ein Imin, auf technisch einfache und wirtschaftliche Weise ermöglicht.

Demgemäß wurde das eingangs definierte Verfahren gefunden.

Als Amine I kommen Hexamethylendiamin oder 6-Aminocapronsäurenitril, sowie deren Gemische in Betracht.

Die Herstellung solcher Amine kann in an sich bekannter Weise erfolgen.

So kann Hexamethylendiamin durch partielle oder vollständige katalytische Hydrierung mit einem molekularen Wasserstoff enthaltenden Gas von Adipodinitril zu Hexamethylendiamin oder Mischungen, die Hexamethylendiamin und 6-Aminocapronsäurenitril enthalten, erhalten werden.

Als Katalysatoren können bei dieser Hydrierung vorteilhaft solche auf der Basis eines Metalles ausgewählt aus der Gruppe bestehend aus Ruthenium, Rhodium, Nickel, Cobalt, vorzugsweise Eisen eingesetzt werden, wobei die Katalysatoren weitere Elemente als Promotoren enthalten können. Im Falle von Katalysatoren auf der Basis von Eisen kommen als Promotoren insbesondere ein oder mehrere, wie zwei, drei, vier oder fünf Elemente ausgewählt aus der Gruppe bestehend aus Aluminium, Silizium, Zirkonium, Titan und Vanadium in Betracht.

Solche Katalysatoren sowie die Verfahrensbedingungen für die genannte Umsetzung sind beispielsweise in WO-A-96/20166, der Deutschen Anmeldung 19 636 768.9 und der Deutschen Anmeldung 19 646 436.6 beschrieben.

Die nach den genannten Verfahren erhaltenen Produkte können anschließend vorteilhaft in Gegenwart von auf Edelmetallen, wie Platin, Palladium oder deren Gemischen, basierenden Katalysatoren mit molekularen Wasserstoff enthaltenden Gasen nachhydriert werden.

Als Imine III kommen Aminohexylidenimin oder Tetrahydroazepin der Formel 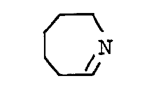 sowie deren Gemische in Betracht.

Die Imine (III) können in der Mischung (II) als einzelne Verbindungen oder als Addukte, beispielsweise an ein Amin (I), vorliegen, wobei diese Addukte im Sinne der vorliegenden Erfindung ebenfalls als Imine (III) bezeichnet werden.

Solche Imine sowie Verfahren zu deren Herstellung sind allgemein bekannt.

So können Aminohexylidenimin und Tetrahydroazepin bei der partiellen katalytischen Hydrierung mit einem molekularen Wasserstoff enthaltenden Gas von Adipodinitril zu Hexamethylendiamin oder Mischungen, die Hexamethylendiamin und 6-Aminocapronsäurenitril enthalten, in der Regel in Mengen von 1 bis 10000 ppm bezogen auf das Gemisch nach den für die Herstellung der Amine (I) beschriebenen Verfahren in Mischungen (II) erhalten werden.

Erfindungsgemäß gibt man dem Destillationsgemisch eine gegenüber dem Amin (I) unter den Destillationsbedingungen inerte Verbindung (IV), deren Siedepunkt unter den Destillationsbedingungen über dem Siedepunkt des Amins (I) liegt, zu.

Als Verbindungen (IV) kommen Verbindungen aus der Gruppe der Aromaten, der Aliphaten, wie acyclische und cyclische Aliphaten, und aliphatisch-aromatische Verbindungen in Betracht. Diese Verbindungen können Substituenten tragen, wie eine Hydroxyl-, Keto-, Ester-, Alkyl-, Aryl, Cycloalkyl-, Arylalkyl-Gruppe, vorzugsweise eine Nitril- oder Amino-Gruppe, oder mehrere gleiche oder unterschiedliche solcher Gruppen.

Die Verbindung (IV) kann aus einer Verbindung oder Gemischen solcher Verbindungen bestehen.

Vorteilhaft sind solche Verbindungen (IV), die sich auf einfache Weise, wie durch Hydrierung beispielsweise mit einem molekularen Wasserstoff enthaltenden Gas in Gegenwart eines Katalysators, zu einer Mischung (V) enthaltend ein Amin (I) und ein Imin (III) oder insbesondere einer Mischung (II) umsetzen lassen.

Die bei dieser Umsetzung erhaltenen Produkte können in das erfindungsgemäße Verfahren vorteilhaft erneut eingesetzt werden.

Die Differenz der Siedepunkte zwischen dem Amin (I) und der Verbindung (IV) sollte unter den Destillationsbedingungen 1 bis 200°C, vorzugsweise 5 bis 100°C betragen.

Setzt man als Amin (I) Hexamethylendiamin und als Imin (III) Aminohexylidenimin, Tetrahydroazepin oder deren Gemische ein, so hat sich der Einsatz von Adipodinitril, 6-Aminocapronsäurenitril oder deren Gemische besonders vorteilhaft erwiesen.

Setzt man als Amin (I) 6-Aminocapronsäurenitril und als Imin (III) Aminohexylidenimin, Tetrahydroazepin oder deren Gemische ein, so hat sich der Einsatz von Adipodinitril oder Mischungen enthaltend im wesentlichen Adipodinitril als besonders vorteilhaft erwiesen.

Die Verbindung (IV) kann zu der Mischung (II) vor oder während der Destillation zugegeben werden.

Die Zugabe der Verbindung (IV) zu der Mischung (II) vor der Destillation kann in an sich bekannter Weise in üblichen Mischapparturen erfolgen.

Die Zugabe der Verbindung (IV) zu der Mischung (II) während der Destillation kann durch Einspeisen der Verbindung (IV) in die Destillationsapparatur vorzugsweise im Sumpfbereich erfolgen.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Die Destillation kann man in mehreren, wie 2 oder 3 Kolonnen, vorteilhaft einer einzigen Kolonne durchführen.

Nach der Destillation erhält man als Sumpfprodukt, eine Mischung (VI) enthaltend im wesentlichen eine Verbindung (IV).

Enthält die Mischung (VI) zusätzlich Amin (I), so kann vorteilhaft die Sumpftemperatur bei der Destillation herabgesetzt werden.

Aus der Mischung (VI) kann in an sich bekannter Weise, beispielsweise durch physikalische Verfahren, wie Destillation oder Extraktion, oder chemische Verfahren, wie Chemisorption oder Hydrierung, die Verbindung (IV) zurückgewonnen werden.

Diese aus der Mischung (VI) erhaltene Verbindung (IV) kann vorteilhaft in die erfindungsgemäße Destillation zurückgeführt werden.

Hexamethylendiamin kann mit Dicarbonsäuren wie Adipinsäure zu technisch bedeutenden Polymeren verarbeitet werden.

## Patentansprüche

1. Verfahren zur destillativen Abtrennung eines Teiles oder der Gesamtheit eines Imins (III), ausgewählt aus der Gruppe bestehend aus Aminohexylidenimin, Tetrahydroazepin oder deren Addukte, aus einer Mischung (II) enthaltend ein Amin (I), ausgewählt aus der Gruppe bestehend aus Hexamethylendiamin und 6-Aminocapronitril und Imin (III), **dadurch gekennzeichnet, daß** man dem Destillationsgemisch eine gegenüber dem Amin (I) unter den Destillationsbedingungen inerte Verbindung (IV), deren Siedepunkt unter den Destillationsbedingungen über dem Siedepunkt des Amins (I) liegt, zugibt und nach der Destillation eine Mischung (VI) als Sumpfprodukt enthaltend im wesentlichen eine Verbindung (IV) erhält.

2. Verfahren nach Anspruch 1, wobei man die Verbindung (IV) der Mischung (II) vor der Destillation zugibt.

3. Verfahren nach Anspruch 1, wobei man die Verbindung (IV) der Mischung (II) während der Destillation zugibt.

4. Verfahren nach den Ansprüchen 1 bis 3, wobei man als Verbindung (IV) eine Verbindung einsetzt, aus der eine Mischung (V), enthaltend ein Amin(I) und ein Imin (III), oder eine Mischung (II) erhalten werden kann.

5. Verfahren nach Anspruch 4, wobei man nach der Destillation die Verbindung (IV) zu einer Mischung (V)enthaltend im wesentlichen ein Amin (I) und ein Imin (III) umsetzt.

6. Verfahren nach den Ansprüchen 1 bis 5, wobei man als Amin (I) Hexamethylendiamin einsetzt.

7. Verfahren nach den Ansprüchen 1 bis 6, wobei man als Verbindung (IV) Adipodinitril, 6-Aminocapronsäurenitril oder deren Gemische einsetzt.

8. Verfahren nach den Ansprüchen 1 bis 7, wobei man aus der Mischung (VI) eine Verbindung (IV) zurückgewinnt.

9. Verfahren nach den Ansprüchen 1 bis 7, wobei man aus der Mischung (VI) eine Verbindung (IV) zurückgewinnt und diese Verbindung (IV) in die Destillation zurückführt.

## Claims

1. A process for distillative separation of part or all of an imine (III) selected from the group consisting of aminohexylideneimine, tetrahydroazepine and the adducts thereof, from a mixture (II) comprising an amine (I) selected from the group consisting of hexamethylenediamine and 6-aminocapronitrile, and said imine (III), which comprises adding to the distillation mixture a compound (IV) which is inert toward said amine (I) under distillation conditions and whose boiling point is above the boiling point of said amine (I) under said distillation conditions to obtain, after the distillation, a mixture (VI) comprising essentially said compound (IV) as bottom product.

2. A process as claimed in claim 1, wherein said compound (IV) is added to said mixture (II) before the distillation.

3. A process as claimed in claim 1, wherein said compound (IV) is added to said mixture (II) during the distillation.

4. A compound as claimed in any of claims 1 to 3, wherein compound (IV) is a compound from which a mixture (V) comprising an amine (I) and an imine (III) or a mixture (II) is obtainable.

5. A process as claimed in claim 4, wherein, after the distillation, said compound (IV) is converted into a mixture (V) comprising essentially an amine (I) and an imine (III).

6. A process as claimed in any of claims 1 to 5, wherein amine (I) is hexamethylenediamine.

7. A process as claimed in any of claims 1 to 6, wherein compound (IV) is adiponitrile, 6-aminocapronitrile or a mixture thereof.

8. A process as claimed in any of claims 1 to 7, wherein a compound (IV) is recovered from said mixture (VI).

9. A process as claimed in any of claims 1 to 7, wherein a compound (IV) is recovered from said mixture (VI) and recycled into the distillation.

## Revendications

1. Procédé de séparation par distillation d'une partie ou de la totalité d'une imine (III), choisie dans le groupe formé par l'aminohexylidène-imine, le tétrahydroazépine ou leurs produits d'addition, dans un mélange (II) contenant une amine (I), choisie dans le groupe formé par l'hexaméthylènediamine et le 6-aminocapronitrile et une imine (III), **caractérisé en ce que** l'on ajoute au mélange de distillation un composé (IV) inerte vis-à-vis de l'amine (I) dans les conditions de la réaction, dont le point d'ébullition est supérieur au point d'ébullition de l'amine (I) dans les conditions de la réaction, et qu'après la distillation on obtient en tant que produit de fond de colonne un mélange (VI) contenant essentiellement un composé (IV).

2. Procédé selon la revendication 1, où l'on ajoute le composé (IV) au mélange (II) avant la distillation.

3. Procédé selon la revendication (I), où l'on ajoute le composé (IV) au mélange (II) pendant la distillation.

4. Procédé selon les revendications 1 à 3, où l'on met en oeuvre en tant que composé (IV) un composé à partir duquel on peut obtenir un mélange (V) contenant une amine (I) et une imine (III), ou un mélange (II).

5. Procédé selon la revendication 4, où l'on convertit après la distillation le composé (IV) en un mélange (V) contenant essentiellement une amine (I) et une imine (III).

6. Procédé selon les revendications 1 à 5, où l'on met en oeuvre comme amine (I) de hexaméthylènediamine.

7. Procédé selon les revendications 1 à 6, où l'on met en oeuvre comme composé (IV) de l'adiponitrile, du nitrile 6-aminocaproïque ou leurs mélanges.

8. Procédé selon les revendications 1 à 7, où l'on récupère du mélange (VI) un composé (IV).

9. Procédé selon les revendications 1 à 7, où l'on récupère du mélange (VI) un composé (IV) et où l'on recycle ce composé (IV) dans la distillation.
